# EUROPÄISCHE PATENTSCHRIFT

(11) **EP 2 719 720 B1**
(45) Veröffentlichungstag und Bekanntmachung des Hinweises auf die Patenterteilung: **12.08.2015**
(21) Anmeldenummer: 13187837.3
(22) Anmeldetag: 09.10.2013
(51) Int. Cl.: C08J 7/06, A41D 19/00, C08F 2/46, C08J 7/12, C08F 8/34, C08K 9/04, C08J 7/14

(54) **Verfahren zur Modifizierung der Oberfläche eines Elastomerproduktes**
Method for modifying the surface of an elastomer product
Procédé destiné à la modification de la surface d'un produit élastomère

(30) Priorität: 09.10.2012 AT 10862012
(43) Veröffentlichungstag der Anmeldung: 16.04.2014
(73) Patentinhaber: Semperit Aktiengesellschaft Holding, 1031 Wien (AT)
(72) Erfinder: Holzner, Armin, 2630 Ternitz (AT); Kern, Wolfgang, 8055 Seiersberg (AT); Lenko, Dietmar, 8051 Graz (AT); Manhart, Jakob Cornelius, 8700 Leoben (AT); Schaller, Raimund, 2620 Neunkirchen (AT); Schlögl, Sandra, 8152 Stallhofen (AT)
(74) Vertreter: Burger, Hannes

(56) Entgegenhaltungen:
- GB-A- 1 116 725
- YUSUF MÜLAZIM ET AL: "Properties of Thiol-ene Photocurable Highly Hydrophobic and Oleophobic Nanocomposite Coatings on ABS and HIPS Substrates", ADVANCES IN POLYMER TECHNOLOGY, Bd. 32, Nr. S1, 13. Juni 2012 (2012-06-13) , Seiten E416-E426, XP055091550, ISSN: 0730-6679, DOI: 10.1002/adv.21289
- SANDRA SCHLÖGL ET AL: "Characteristics of the photochemical prevulcanization in a falling film photoreactor", JOURNAL OF APPLIED POLYMER SCIENCE, Bd. 124, Nr. 4, 15. Mai 2012 (2012-05-15), Seiten 3478-3486, XP055093985, ISSN: 0021-8995, DOI: 10.1002/app.35457

## Beschreibung

Die Erfindung betrifft ein Verfahren zur Modifizierung der Oberfläche eines Elastomerhandschuhs mit ungesättigten Kohlenstoff-Kohlenstoff-Bifidungen, sowie einer Hanolschuh, mit ungesättigten Kohlenstoff-Kohlenstoff-Bindungen und mit einer Oberfläche.

Die Modifizierung der Oberfläche von Naturkautschukhandschuhen ist aus dem Stand der Technik bereits bekannt. Beispielsweise wird die Oberfläche mit Beschichtungen versehen oder aufgeraut, um damit eine bessere Gleitfähigkeit der Handschuhe zu erreichen. Insbesondere soll damit die Anziehbarkeit bzw. die Nassanziehbarkeit der Handschuhe verbessert werden. Es sind auch Funktionalisierungen zur Reduktion des Allergiepotentials, das Naturkautschuk anhaftet, bekannt.

Aus dem Stand der Technik ist weiter die UV-Vernetzung von Naturkautschuk über die Thiol-En Reaktion bekannt, beispielsweise aus den auf die Anmelderin zurückgehenden Druckschriften AT 502 764 A1 und AT 508 099 A1.

Druckschrift GB 1 116 725 A betrifft ein Verfahren zur Oberflächenbehandlung eines durch Vinylpolymerisation hergestellten Polymerisats, in dem ein Film aus Organosiliziumverbindungen auf die Oberfläche des Polymerisats aufgetragen und mit dieser durch Strahlung verbunden wird. Insbesondere zeigt das Dokument, dass die ungesättigten Kohlenstoff-Kohlenstoff-Bindungen mit einem Mercaptan gesättigt werden.

Der vorliegenden Erfindung liegt die Aufgabe zugrunde, eine alternative Möglichkeit zur Oberflächenmodifizierung von Elastomerhandschuhen.

Gelöst wird diese Aufgabe einerseits indem bei dem eingangs genannten Verfahren die ungesättigten Kohlenstoff-Kohlenstoff-Bindungen im Bereich der Oberfläche zumindest teilweise durch eine photochemische Reaktion mit zumindest einem Thiol gesättigt werden oder indem auf die Oberfläche des Elastomerhandschuhs zumindest bereichsweise eine Schicht aus einem Latex aufgetaucht wird, dessen ungesättigten Kohlenstoff-Kohlenstoff-Bindungen im Bereich seiner Oberfläche zumindest teilweise durch eine photochemische Reaktion mit zumindest einem Thiol gesättigt sind bzw. bei dem eingangs genannten Elastomerhandschuhs die ungesättigten Kohlenstoff Kohlenstoff-Bindungen an der Oberfläche zumindest teilweise durch-SR Gruppen funktionalisiert sind, wobei R für zumindest ein Element steht, das ausgewählt ist aus einer Gruppe umfassend H, Vinylverbindungen, Acrylate, Amine, Aminosäuren (Cystein), acetylierte Aminosäuren (N-Acetylcystein), Anhydride, Carbonsäuren, Ether, Epoxide, Isocyanate, Isothiocyanate, Methacrylate, Silane, Siloxane, Feststoffpartikel, Polymerbeschichtungen.

Von Vorteil ist dabei, dass die photochemische Reaktion über den Thiol-En Reaktionsmechanismus abläuft, sodass mit dem Verfahren eine hohe Effizienz der Umsetzung erreicht werden kann. Darüber hinaus ist dieses Verfahren einfach mit relativ hoher Reaktionsgeschwindigkeit durchführbar. Bedingt durch die photochemische Aktivierung besteht darüber hinaus die Möglichkeit, dass die Absättigung der ethylenischen Doppelbindungen nur in selektiven, vordefinierbaren Teilbereichen der Oberfläche erfolgt, wodurch eine bewusste Oberflächenstrukturierung in diesen Bereichen erreicht werden kann. Darüber hinaus wird durch die Absättigung der ethylenischen Bindungen die Klebrigkeit der Oberfläche des Elastomerhandschuhs verringert. Gleichzeitig kann damit auch die Alterungsbeständigkeit des Elastomers verbessert werden. Ein weiterer Vorteil dieses Verfahrens ist darin zu sehen, dass diese photochemische Reaktion bei Raumtemperatur abläuft, also keine Erwärmung erforderlich ist.

Nach einer Ausführungsvariante des Verfahrens ist vorgesehen, dass durch die Realtion mit dem zumindest einem Thiol freie -SH Gruppen auf der Oberfläche des Elastomerhandschuhs erzeugt werden. Es können mit dieser Ausführungsvariante reaktive Stellen auf der Elastomeroberfläche geschaffen werden. Dadurch wird es möglich, die so vorbereitete Oberfläche weiter zu modifizieren, beispielsweise wiederum über eine Thiol-En Reaktion mit einer vinylischen Verbindung, insbesondere nach einem photochemischen Reaktionsmechanismus. Es kann damit das Spektrum der möglichen Oberflächenmodifizierungen vergrößert werden, wobei der Vorteil erreicht wird, dass die Anbindung von weiteren Substanzen an die Elastomeroberfläche kovalent erfolgt, sodass die Haftung im Vergleich mit rein adhäsiv gebundenen Substanzen deutlich besser ist. Insbesondere kann also damit vermieden werden, dass die an die Oberfläche angebundenen Substanzen während des Gebrauchs des Elastomers abgegeben werden.

Vorzugsweise wird dazu als Thiol eine Mercaptoverbindung verwendet, die ausgewählt ist aus einer Gruppe umfassend bzw. bestehend aus Trimethylolpropan-tris-3-mercaptopropionat, Pentaerythritoltetramercaptoacetat, Trimethylolpropantrimercaptoacetat, Trimethylolpropanetri-3-mercaptopropionat, Pentaerythritoltetra-3-mercaptopropionat, Propylenglycol-3-mercaptopropionat, ethoxyliertes Trimethylolpropantri-3-mercaptopropionat, Polyol-3-mercaptopropionat, Polyester-3-mercaptopropionat. Diese Verbindungen sind beispielsweise bei Bruno Bock Thiochemicals und/oder Sigma Aldrich erhältlich. Durch den Einsatz dieser Thiole wird der Vorteil erreicht, dass eine höhere Funktionalität vorhanden ist, sodass Ankergruppen für weitere Reaktionen zur Verfügung stehen. Darüber hinaus sind diese Verbindungen nicht toxisch und nicht karzinogen.

Vorzugsweise wird das Elastomerhandschuh vorvernetzt eingesetzt. Es kann damit eine Reduktion der eingesetzten Chemikalien erreicht werden. Andererseits ist damit aber auch eine Erhöhung der Produktionsgeschwindigkeit erzielbar. Zudem kann eine Erhöhung der Härte des Elastomers vermieden werden, die gegebenenfalls aufgrund von sterischen Behinderungen in der Masse des Elastomers auftreten können.

Gemäß einer Ausführungsvariante dazu ist vorgesehen, dass die Vorvernetzung ebenfalls photochemisch durchgeführt wird. Es wird damit der Vorteil der Kontinuität der eingesetzten Methoden im Gesamtprozess zur Herstellung der Elastomerhandschuhe erreicht.

Die Reaktion mit dem zumindest einem Thiol kann an einer festen Oberfläche des Elastomerhandschuhs durchgeführt werden. Diese Verfahrensvariante wird insbesondere zur Herstellung von einschichtigen Elastomerhandschuhen eingesetzt, da es damit möglich ist, gezielt Oberflächenbereiche des Elastomerhandschuhs zu modifizieren.

Es kann weiter vorgesehen werden, dass die freien -SH Gruppen mit zumindest einer weiteren chemischen Verbindung und/oder mit Feststoffpartikeln umgesetzt werden. Es kann also im Rahmen der Erfindung eine weitere Funktionalisierung bzw. eine andere Funktionalisierung - bezogen auf die Oberflächenmodifizierung mittels Thiolen - erreicht werden, wodurch das Einsatzgebiet der Elastomerhandschuhe vielfältiger und an unterschiedlichste Anforderungen ausgedehnt werden kann.

Die weitere chemische Verbindung kann dabei ausgewählt werden aus einer Gruppe umfassend bzw. bestehend aus Alkene, Acrylate, Anhydride, Epoxide, Isocyanate, Isothiocyanate, Methacrylate, Thiole. Mit diesen Verbindungen wird der Vorteil erreicht, dass maßgeschneiderte Ankergruppen für unterschiedliche weitere Reaktionen, auch thermische Reaktionen, zur Verfügung gestellt werden können. Es ist damit möglich, die Oberflächenpolarität und die Gleitreibungseigenscbanen des Elastomerhandschuhs gezielt zu verändern.

Vorzugsweise sind die Feststoffpartikel durch anorganische Partikel gebildet. Es kann damit eine Reduktion der Klebrigkeit bzw. eine Verbesserung der Anziehbärkeit, insbesondere der Nassanziehbarkeit von Handschuhen erreicht werden, indem die Kontaktfläche des Elastomers mit einer Hand reduziert wird. Generell kann damit die Haftung eines Elastomerhandschuhs an einer Oberfläche aufgrund dieses Effektes reduziert werden. Darüber hinaus ist es damit aber auch möglich, dass über diese Feststoffpartikel eine zusätzliche Funktionalität in das Elastomerprodukt eingebracht wird, beispielsweise wenn Feuchtigkeit absorbierende Feststoffpartikel verwendet werden.

Zur besseren Anbindung der Feststoffpartikel an die funktionalisierte Oberfläche des Elastomerhandschuhs ist es von Vorteil, wenn die Feststoffpartikel vor der Reaktion mit den-SH Gruppen ebenfalls oberflächlich funktionalisiert werden.

Die Funktionalisierung der Feststoffpartikel kann dabei durch Erzeugung von freien Epoxygruppen, Mercaptogruppen, Acrylatgruppen, Anhydridgruppen, Isocyanatgruppen, Isothiocyanatgruppen, Methacrylatgruppen, Vinylgruppen, an der Oberfläche der Feststoffpartikel erfolgen. Durch den Einsatz dieser funktionellen Gruppen wird der Vorteil erreicht, dass diese kovalent an die Elastomeroberfläche angebunden werden können, womit eine geringere Gefahr der Wundkontamination und ein niedrigeres Allergiepotential erreicht werden. Zudem können damit für Reinräume geeignete Handschuhe zur Verfügung gestellt werden.

Die Funktionalisierung der Feststoffpartikel kann aber auch mit zumindest einer chemischen Verbindung durchgeführt werden, die ausgewählt ist aus einer Gruppe umfassend oder bestehend aus Silanen, Siloxanen und Carbonsäuren mit funktionellen Gruppen, wie Acrylat-, Anhydrid-, Epoxy-, Isocyanat-, Isothiocyanat-, Mercapto-, Methacrylat-, Vinylgruppen. Beispiele hierfür sind Vinyltriethoxysilan, (3-Glycidoxypropyl)trimethoxysilan, 3-(Triethoxysilyl)propylsuccinic Anhydride, Mercaptopropyltrimethoxysilan. Diese Verbindungen sind erhältlich bei ABCR bzw. Sigma Aldrich.

Um so genannte "puderfreie" Handschuhe, bereit zu stellen, ist nach einer anderen Ausführungsvariante vorgesehen, dass rein adhäsiv gebundene Partikel von der Oberfläche des Elastomerhandschuhs entfernt werden. Es kann damit das Allergiepotential der Elastomerprodukte reduziert werden. Zudem können damit diese adhäsiv gebundenen Partikel, die im Vergleich zu den kovalent gebundenen Partikeln eine geringere Wirkung haben, gegebenenfalls in den Produktionsprozess zurückgeführt werden.

Neben der Ausführungsvariante des Verfahrens, nach der die Funktionalisierung an einer festen Oberfläche des Elastomerhandschuhs durchgeführt wird, besteht im Rahmen der Erfindung auch die Möglichkeit, dass die photochemische Reaktion mit zumindest einem Thiol an einem Latex in flüssiger Phase durchgeführt wird und der Latex danach auf einen, insbesondere vorvernetzten, Latexfilm aufgetaucht wird. Diese Verfahrensvariante wird insbesondere für die Herstellung mehrschichtiger Elastomerprodukte verwendet. Der Vorteil dabei ist, dass mit dieser Verfahrensvariante bei Bedarf Thiole nicht nur im Bereich der Oberfläche an einem Film kovalent angebunden werden können sondern bereits an den einzelnen Latexpartikel, wodurch eine Anpassung des Eigenschaftspotentials des Elastomerproduktes erfolgen kann.

Es ist weiter möglich, dass die Oberflächenmodifizierung nur in diskreten Bereichen des Elastomerproduktes durchgeführt wird. Es kann damit eine stärkere Strukturierung der Elastomeroberfläche erreicht werden, wodurch die Gleitfähigkeit des Elastomerproduktes beeinflusst werden kann. Zudem können bestimmten Bereichen des Elastomerproduktes spezifische Eigenschaften verliehen werden, indem in den modifizierten Bereichen in weiterer Folge weitere chemische Verbindungen an die Thiolgruppen kovalent angebunden werden.

Nach einer anderen Verfahrensvariante ist vorgesehen, dass auf die Oberfläche des Elastomerproduktes eine Polymerbeschichtung aufgebracht wird. Es kann damit diese Polymerbeschichtung über die Thiolgruppen kovalent an die Elastomeroberfläche angebunden werden, wodurch eine bessere Haftung der Polymerbeschichtung an der Elastomeroberfläche erreicht wird.

Zum besseren Verständnis der Erfindung wird diese anhand der nachfolgenden Figuren näher erläutert.

Es zeigen teilweise in schematisch vereinfachter Darstellung:
- Fig. 1: die Modifizierung von NR-Latexfilmen mit vinylfunktionalisierten SiO₂-Partikeln;
- Fig. 2: die strukturierte Belichtung von Polymeren;
- Fig. 3: den Vergleich der IR-Spektren von unbehandelten und mit TriThiol modifizierten Polyisoprenfilmen;
- Fig. 4: die Änderung des Kontaktwinkels von Wasser durch eine Oberflächerifunktionalisierung mittels TriThiol.

Im Rahmen der Erfindung wurden unterschiedliche Ansätze entwickelt, um die Klebrigkeit von Kautschukfilmen zu verringern bzw. generell die Eigenschaften von Elastomerprodukten zu verbessern bzw. zu verändern.

Unter einem Elastomerprodukt wird ein Handschuh, vorzugsweise ein chirurgischer Handschuh oder ein Untersuchungshandschuh verstarkten.

Der Vollständigkeit halber sei angemerkt, dass unter einem Elastomerhandschuh im Rahmen der Erfindung ein Produkt aus einem Elastomer verstanden wird, das in der Molekülstruktur ungesättigte Kohlenstoff-Kohlenstoff Bindungen aufweist, also insbesondere ethylenische Bindungen (=Dienkautschuk). Vorzugsweise ist das Elastomer ein Naturkautschuk oder ein synthetischer Isoprenkautschuk. Daneben ist die Erfindung auch auf andere derartige, ungesättigte Kohlenstoff-Kohlenstoff Bindungen aufweisende Kautschukarten anwendbar, insbesondere insbesondere Homopolymere und Copolymere wie Nitril-Butadien-Kautschuk, carboxylierter Nitril-Butadien-Kautschuk, Polybutadien, Polychloropren, Styrol-Butadien-Kautschuk.

Das Tauchverfahren zur Herstellung von Gummibandschuhen ist im Stand der Technik bereits ausführlich beschrieben. Üblicherweise umfasst es zumindest folgende Schritte: Bereitstellen einer Tauchform, Auftauchen eines Koagulanten, Auftauchen eines Latex. Daneben umfasst dieses Tauchverfahren diverse Wasch- und Trockenschritte. Üblicherweise wird dieses Täuchverfähren kontinuierlich durchgeführt, beispielsweise in einer so genannten Kettentauchanlage. Für weitere Einzelheiten dazu sei auf den einschlägigen Stand der Technik verwiesen.

Sämtlichen Ausführungsvarianten der Erfindung ist gemein, dass die ungesättigten Kohlenstoff-Kohlenstoff Bindung zumindest im Bereich der Oberfläche des Elastomerhandschuhs (im Folgendem wird nur mehr auf ein Elastomer Bezug genommen, wobei diese Bezugnahme auch das Elastomerprodukt einschließt) zumindest teilweise, vorzugsweise zu zumindest annähernd 2 %, insbesondere zwischen 3 % und 75 %, vorzugsweise zwischen 4 % und 10 %, durch Reaktion mit einer Mercaptogruppe abgesättigt werden.

Die Mercaptogruppe wird insbesondere in Form eines Thiols zur Verfügung gestellt. Bevorzugt werden dazu Thiole verwendet, die ausgewählt sind aus einer Gruppe umfassend oder bestehend aus Trimethylolpropan-tris-3-mercaptopropionat, 16-Mercaptohexadecansäure, (11-Mercaptoundecyl)tetra(ethylenglycol), N-Acetyl-L-cystein, Pentaerythritoltetramercaptoacetat, Trimethylolpropantrimercaptoacetat, Trimethylolpropanetri-3-mercaptopropionat, Pentaerythritoltetra-3-mercaptopropionat, Propylenglycol-3-mercaptopropionat, ethoxyliertes Trimethylolpropantri-3-mercaptopropionat, Polyol-3-mercaptopropionat, Polyester-3-mercaptopropionät.

Neben diesen bevorzugt verwendeten chemischen Verbindungen mit einer Mercaptogruppe können im Rahmen der Erfindung auch andere derartige Verbindungen eingesetzt werden, wie beispielsweise HS-R1R2R3, wobei R1 durch ein Element aus der Gruppe umfassend oder bestehend aus Alkyl-, Aryl-, Alkylaryl-, Arylalkyl-, Alkylarylalkyl-, Arylalkylaryl-, Silylgruppen, R2 durch ein Element aus der Gruppe umfassend oder bestehend aus Acryl-, Amino, Aminosäure-, Anhydrid-, Carbonyl- (C=O), Carbonsäure-, Carboxylat,- Epoxy-, Hydroxy-, Isocyanat-, Isothiocyanat-, Methacryl-, Mercapto-, Sulfonsäure-, Vinyl- gruppen, R3 durch ein Element aus der Gruppe umfassend oder bestehend aus H, Alkyl-, Aryl-gruppen gebildet sind. Beispiele hierfür sind Poly(ethylenglycol)methyl ether thiol, 11-Mercapto-1-undecanol, 16-Mercaptohexadecansäure, Cysteamin, Cystein, 2-Propen-1-thiol, cis-9-Octadecen-1-thiol.

Der Einsatz von mehrfach funktionellen Thiolen, d.h. von chemischen Verbindungen mit mehr als einer Mercaptogruppe, wie beispielsweise das voranstehend genannte Trimethylolpropan-tris-3-mercaptopropionat hat den Vorteil, dass damit freie Mercaptogruppen zumindest an der Oberfläche des des Elastomerhandschuhs erzeugt werden können, über die eine weitere Reaktion mit weiteren chemischen Verbindungen zur weiteren Veränderung der Eigenschaften des des Elastomerhandschuhs ermöglicht wird.

Neben der Ausführungsvariante mit chemischen Verbindungen mit mehrfacher Homofunktionalität, also mit Verbindungen die ausschließlich Mercaptogruppen als funktionelle Gruppen im Molekül aufweisen, besteht auch die Möglichkeit, mehrfach funktionelle chemische Verbindung mit Heterofunktionalität einzusetzen. Bei diesen Verbindungen ist neben zumindest einer Mercaptogruppe, über die die Anbindung der Verbindung an die Oberfläche des Elastomerhandschuhs erfolgt, zumindest eine weitere Funktionalität vorhanden, beispielsweise eine Aminogruppe, eine Carbonsäuregruppe, eine Acrylat-, Anhydrid-, Epoxy-, Isocyanat-, Isothiocyanat-, Methacrylat-, Vinylgruppe, wobei auch Mischvarianten möglich sind, dass also mehr als eine dieser Gruppen zusätzlich zu der oder den Mercaptogruppen vorhanden sind, beispielsweise eine Carbonsäuregruppe und eine Aminogruppe oder eine Acrylatgruppe mit einer Anhydridgruppe, etc..

Prinzipiell bestehen zwei Verfahrensvarianten. Zum einen ist es möglich, die Funktionalisierung mit dem zumindest einen Thiol - es können auch Mischungen von mehreren unterschiedlichen Thiolen verwendet werden - an einer festen Oberfläche des des Elastomerhandschuhs durchzuführen. Zum anderen besteht die Möglichkeit, dass die Funktionalisierung in der Flüssigphase des Latex erfolgt, und danach eine entsprechende Form in den Latex getaucht und damit der Elastomerartikel hergestellt wird.

Bei der Ausführungsvariante des Verfahrens an der festen Oberfläche des Elastomers ist es nicht zwingend notwendig, dass der Elastomerhandschuh nach einem Tauchverfahren hergestellt wird. Es sind alle formgebenden Verfahren, die aus dem Stand der Technik bekannt sind, beispielsweise Spritzgussverfahren, Extrusionsverfahren, Compression Molding, etc. einsetzbar, wenngleich das Tauchverfahren im Rahmen der Erfindung das bevorzugte Verfahren zur Herstellung des Elastomerproduktes ist.

Für die Absättigung bzw. Reaktion der ungesättigten Kohlenstoff-Kohlenstoff Bindungen au einer festen Oberfläche eines Elastomerhandschuhs wird die Elastomeroberfläche mit dem jeweiligen Reagens in Kontakt gebracht. Das Elastomer wird bevorzugt vorvernetzt eingesetzt, wobei die Vorvernetzung bevorzugt photochemisch mit einem Thiol durchgeführt wird, wie dies in den Druckschriften AT 502 764 A1 und AT 508 099 A1 beschrieben ist. Generell wird im Rahmen der Erfindung die Vorvernetzung bevorzugt photochemisch mit einem Thiol durchgeführt. Der Vollständigkeit halber sei angeführt, dass auch andere, bekannte Vernetzungsverfahren verwendet werden können, beispielsweise kann eine peroxidische Vorvernetzung oder eine Salzbadvernetzung oder eine Vernetzung mittels aktinischer Strahlung durchgeführt werden. Ebenso kann eine Schwefelvemetzung (bei erhöhter Temperatur), wie sie prinzipiell aus dem Stand der Technik bekannt ist, als Vorvernetzung durchgeführt werden.

Das jeweilige Reagens, also ein ein- oder mehrfach funktionelles Thiol wird vorzugsweise als Emulsion eingesetzt. Insbesondere wird eine wässrige Emulsion verwendet, wobei prinzipiell auch Emulsionen mit organischen Lösungsmitteln verwendbar sind.

Die Konzentration des zumindest einen Thiols in der Emulsion kann zwischen 1 Gew.-% und 20 Gew.-%, insbesondere zwischen 1 Gew.-% und 10 Gew.-%, betragen.

Zudem kann die Emulsion auch diverse Hilfsmittel, wie z.B. Emulgatoren, beispielsweise TWEEN^{®} 20, oder Stabilisatoren, Antioxidantien, Farbstoffe, Antiozonantien enthalten. Der Summenanteil an dem oder den zugesetzten Emulgator(en) beträgt vorzugsweise zwischen 0, 5 Gew.-% und 5 Gew.-%, bezogen auf die Gesamtmasse der Emulsion.

Nachdem die Reaktion photochemisch durchgeführt wird, insbesondere bei einer Wellenlänge bzw. einem Wellenlängenspektrum im sichtbaren und/oder UV-Bereich, wird der Emulsion vorzugsweise auch zumindest ein Photoinitiator zugesetzt, beispielsweise Lucirin^{®} TPO L. Weitere verwendbare Photoinitiatoren sind in der AT 502 764 A1 und der AT 508 099 A1 beschrieben, auf die diesbezüglich Bezug genommen wird.

Der Summenanteil an dem oder den zugesetzten Photoinitiator(en) beträgt vorzugsweise zwischen 0,5 Gew.-% und 5 Gew.-%, bezogen auf die Gesamtmasse der Emulsion.

Sofern ein wasserlösliches Thiol verwendet wird, besteht die Möglichkeit, dass dieses in dem Wasser gelöst wird. Ebenso können flüssige Reinsubstanzen eingesetzt werden. In diesem Fall beträgt der Summenanteil an wasserlöslichem/n Thiol(en) zwischen 1 Gew.-% und 25 Gew.-%.

Zur Herstellung der Emulsion können handelsübliche Dispergiergeräte verwendet werden.

Die Emulsion bzw. die Aufbereitung mit dem zumindest einen Thiol wird in der Folge mit dem Elastomer in Kontakt gebracht, beispielsweise indem das Elastomer in die Emulsion getaucht wird. Die Temperatur dabei kann zwischen 10 °C und 70 °C betragen. Weiter kann die Dauer der "Benetzung" zwischen 0,1 Minuten und 60 Minuten betragen. Danach wird das benetzte Elastomer getrocknet.

Es ist auch möglich, dass die Benetzung des Elastomers mit der Thiolaufbereitung in mehreren Schritten erfolgt, wobei gegebenenfalls zwischen den einzelnen Schritten eine Zwischentrocknung erfolgt.

Zur Ausbildung der kovalenten Bindungen zwischen dem Elastomer und dem zumindest einen Thiol wird das benetzte Elastomer mit einer geeigneten Strahlungsquelle belichtet, beispielsweise mit einer UV-Strahlungsquelle, wie z.B. einem Hg-Strahler (nicht dotiert oder dotiert, beispielsweise mit Gallium oder Eisen dotiert) oder einem Laser.

Die Bestrahlungsdosis kann zwischen 0,2 J/cm² und 50 J/cm², insbesondere zwischen 0,5 J/cm² und 10 J/cm² betragen.

Die Temperatur dabei kann zwischen 10 °C und 100 °C betragen.

Gegebenenfalls kann die Belichtung in mehreren Schritten durchgeführt werden, beispielsweise in zwei bis acht Wiederholungen.

Nach der Belichtung kann das so behandelte Elastomer noch gewaschen, beispielsweise mit Wasser und/oder einem organischen Lösungsmittel, und/oder getrocknet werden.

Durch diese Behandlung des Elastomers wird eine Reduktion der Klebrigkeit, d.h. eine Erhöhung der Gleitfähigkeit, und eine Verbesserung der Alterungsbeständigkeit erreicht.

Bei Verwendung von mehrfach funktionellen Thiolen können damit auch reaktive Gruppen an der Oberfläche des Elastomers erzeugt werden, beispielsweise weitere Thiolgruppen oder Aminogruppen oder Carbonsäuregruppen, wie dies voranstehend bereits ausgeführt wurde. Diese reaktiven Gruppen können dazu verwendet werden, um das funktionalisierte Elastomer mit weiteren chemischen Verbindungen zu versehen, die mit diesen Gruppen reagieren können.

So ist nach einer Ausführungsvariante des Verfahrens vorgesehen, dass die freien -SH Gruppen und/oder die anderen genannten reaktiven Gruppen mit zumindest einer weiteren chemischen Verbindung umgesetzt werden.

Die weiteren chemischen Verbindungen können ausgewählt werden aus einer Gruppe umfassend Thiole, Epoxide und Isocyanate. Beispielsweise kann eine thermische Anbindung (es ist generell eine Mischvariante einer photochemischen mit einer thermischen Reaktion möglich) von Thiolen unter Disulfidbildung durchgeführt werden. Epoxide können unter Ringöffnung reagieren. Durch die Ankopplung von weiteren chemischen Verbindungen kann eine Änderung der Oberflächenpolarität, eine erhöhte Alterungsbeständigkeit durch eine Absättigung der C=C Doppelbindung erreicht werden bzw. kann damit eine anderen Oberflächenreaktivität erreicht werden.

Diese Umsetzung kann je nach Reaktionspartner bei einer Temperatur zwischen 10 °C und 120 °C und nach bekannten Reaktionsmechanismen erfolgen. Die Dauer dieser Umsetzung richtet sich ebenfalls nach den jeweiligen konkreten Verbindungen die zur Reaktion gebracht werden und kann zwischen 1 Minute und 90 Minuten betragen. Gegebenenfalls kann die Umsetzung unter Druck oder unter Vakuum durchgeführt werden. Ebenso ist aktinische Strahlung verwendbar.

Mit dem Verfahren nach der Erfindung ist es aber auch möglich Feststoffpartikel, vorzugsweise großtechnische verfügbare Feststoffpartikel, insbesondere anorganische Feststoffpartikel, kovalent, insbesondere ausschließlich kovalent, an die Elastomeroberfläche zu binden. Diese Feststoffpartikel können vorzugsweise ausgewählt sein aus einer Gruppe umfassend oder bestehend aus Sulfiden, Oxiden, Hydroxiden, Carbonaten, Boraten, Sulfaten, Phosphaten, Silikaten, Metallpartikel, z.B. Gold, Silber, Kupfer. Insbesondere sind die Feststoffpartikel ausgewählt aus einer Gruppe umfassend oder bestehend aus Kreide, Kieselgur, Kieselerde, Kaolinite, Quarz, amorphe Kieselsäure, SiO₂, Calcit, TiO₂.

Es können aber auch organische Feststoffpartikel, beispielsweise zumindest teilweise bestehend aus Stärke oder Cellulose, kovalent an die Elastomeroberfläche angebunden werden.

Es können auch Hohlräume aufweisende Partikel, die gegebenenfalls mit einem Wirkstoff beladen sind, beispielsweise Zeolithe oder Cyclodextrine, eingesetzt werden. Gegebenenfalls können diese Partikel auch zur Adsorption von Stoffen, wie z.B. Schweiß, eingesetzt werden.

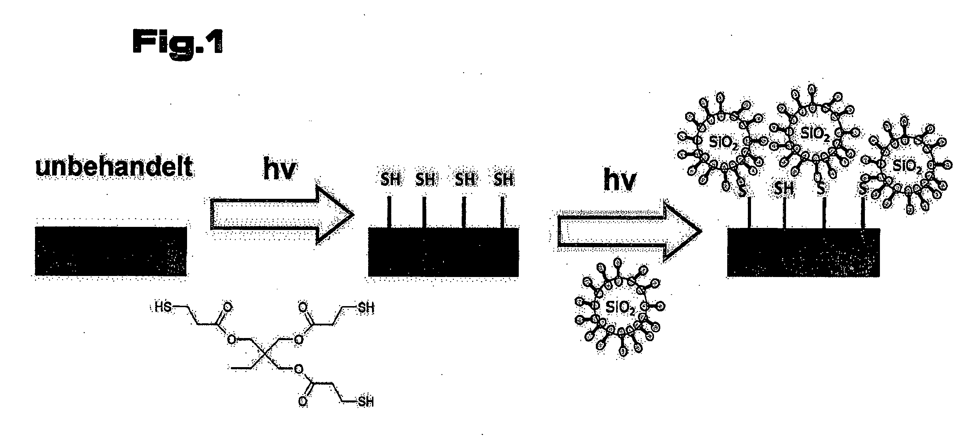

Vorzugsweise erfolgt auch diese Anbindung von Feststoffpartikel an die Elastomeroberfläche photochemisch unter den voranstehend beschriebenen Bedingungen. Die Umsetzung selbst kann sowohl in wässrigen Medien als auch in flüssigen organischen Medien erfolgen. Ein möglicher schematischer Verfahrensablauf ist in Fig. 1 dargestellt, wobei als Feststoffpartikel vinylfunktionalisierte SiO₂-Partikel dargestellt sind.

Durch diese Vorgangsweise wird der Vorteil erreicht, dass ein quantitatives Entfernen von nicht kovalent gebundenen Partikeln möglich ist, da die Klebrigkeit der Elastomeroberfläche im ersten Schritt durch die Absättigung der ungesättigten Kohlenstoff-Kohlenstoff Bindungen deutlich verringert wird.

Im speziellen Fall nach Fig. 1 wurde ein tri-funktionelles Thiol (TriThiol) über die UV initiierte Thiol-En Reaktion an die NR-Oberfläche gekoppelt. Durch diese Modifizierung wird ein Großteil der C=C-Doppelbindungen abgesättigt und der Film verliert seine Klebrigkeit. An die freien SH-Gruppen werden über eine zweite Thiol-En Reaktion vinylfunktionalisierte SiO₂-Partikel an die Oberfläche gebunden.

Von Vorteil ist dabei, wenn die Feststoffpartikel vor der Reaktion mit den -SH Gruppen der Elastomeroberfläche oberflächlich funktionalisiert werden. Diese Funktionalisierung kann beispielsweise durch Erzeugung von freien Epoxygruppen, Mercaptogruppen, Acrylätgruppen, Anhydridgruppen, Isocyanatgruppen, Isothiocyanatgruppen, Methacrylatgruppen, Vinylgruppen, an der Oberfläche der Feststoffpartikel durchgeführt werden. Insbesondere kann dazu eine chemische Verbindung verwendet werden, die ausgewählt ist aus einer der voranstehenden Gruppen.

Die Anbindung der Feststoffpartikel an die Elastomeroberfläche kann entsprechend dem allgemeinen, voranstehend dargestellten Verfahrensablauf über die Mercaptogruppen und/oder weitere funktionelle Gruppe an der Elastomeroberfläche, wie beispielsweise die voranstehend genannten funktionellen Gruppen, und insbesondere photochemisch erfolgen.

Es ist aber auch möglich, die Feststoffpartikel thermisch an die Elastomeroberfläche anzukoppeln, d.h. kovalent anzubinden. Dazu werden vorzugsweise mit Epoxygruppen und/oder Aminogruppen oberflächlich modifizierte Feststoffpartikel eingesetzt. Die Herstellung dieser modifizierten Feststoffpartikel kann durch Umsetzung der Feststoffpartikel mit den voranstehend genannten Verbindungen unter den voranstehend genannten Bedingungen erfolgen.

Die funktionalisierten Feststoffpartikel werden in Wasser oder einem organischen Lösungsmittel suspendiert. Danach wird diese Suspension mit der funktionalisierten Elastomeroberfläche in Kontakt gebracht, beispielsweise durch Eintauchen des Elastomers in die Suspension. Gegebenenfalls kann dies mehrmals durchgeführt werden.

Anschließend wird das so behandelte Elastomer getrocknet. Die Temperatur kann dabei ausgewählt sein aus einem Bereich von 40 °C bis 150 °C, insbesondere aus einem Bereich von 40 °C bis 100 °C. Die Trocknung kann während einer Zeitspanne zwischen 5 Minuten und 1000 Minuten, insbesondere während einer Zeitspanne zwischen 10 Minuten und 900 Minuten, erfolgen.

Während der Trocknung erfolgt die thermische Anbindung der Feststoffpartikel an die Elastomeroberfläche.

Es ist auch möglich, die voranstehend genannte weitere Funktionalisierung der funktionalisierten Elastomeroberfläche mit zumindest einer weiteren chemischen Verbindung thermisch unter diesen Bedingungen durchzuführen.

Bevorzugt werden nach der Anbindung rein adhäsiv gebundene Partikel von der Oberfläche des Elastomerproduktes entfernt, beispielsweise durch Waschen und/oder mechanisch, beispielsweise mittels Ultraschall.

Die im Rahmen der Erfindung verwendeten Feststoffpartikel haben vorzugsweise eine Partikelgröße zwischen 0,01 µm und 1000 µm, insbesondere zwischen 0,1 µm und 10 µm.

Nach einer anderen Verfahrensvariante ist vorgesehen, dass die photochemische Reaktion mit zumindest einem Thiol an einem Latex in flüssiger Phase durchgeführt wird und der Latex danach auf einen, insbesondere vorvernetzten, vorzugsweise photochemisch vorvernetzten, Latexfilm aufgetaucht wird.

Dazu wird das zumindest eine Thiol in einem Lösungsmittel, insbesondere Wasser, wobei auch organische Lösungsmittel verwendet werden können, gelöst. Das zumindest eine Thiol kann aus den voranstehend genannten Thiolen ausgewählt sein. Bevorzugt wird N-Acetylcystein und/oder Cystein und/oder ethoxyliertes Trimethylolpropan-tri-3-mercaptopropionat verwendet.

Die Lösungsmittelmenge wird vorzugsweise dabei so bemessen, dass sich nach der Latexzugabe ein Feststoffgehalt zwischen 15 % drc (dry rubber content) und 40 % drc, insbesondere zwischen 20 % drc und 30 % drc, einstellt.

Der Anteil an dem zumindest einen Thiol an der Lösung ohne Latex kann zwischen 0,5 phr und 50 phr, insbesondere zwischen 1 phr und 20 phr, betragen.

Neben dem zumindest einen Thiol enthält die Lösung vorzugsweise noch zumindest einen Photoinitiator. Mögliche Photoinitiatoren sind in der AT 502 764 A1 und der AT 508 099 A1 beschrieben, auf die diesbezüglich Bezug genommen wird.

Der Summenanteil des zumindest einen Photoinitiators kann ausgewählt sein aus einem Bereich von 0,5 phr bis 5 phr, insbesondere aus einem Bereich von 1 phr bis 2 phr, bezogen auf den Latex.

Das Lösen der Inhaltsstoffe der Lösung ohne Latex kann bei einer Temperatur zwischen 10 °C und 50 °C und/oder für eine Zeit zwischen 0,5 Minuten und 60 Minuten erfolgen.

Diese Lösung wird mit dem zugesetzten Latex in der Folge photochemisch umgesetzt, vorzugsweise mit einer Wellenlänge oder einem Wellenlängenspektrum aus dem sichtbaren und/oder UV-Bereich. Die Strahlungsquelle kann aus den voranstehend genannten Strahlungsquellen ausgewählt sein.

Die Bestrahlungsdosis kann zwischen 0,2 J/cm² und 50 J/cm², insbesondere zwischen 0,5 J/cm² und 10 J/cm² betragen.

Die Temperatur dabei kann zwischen 10 °C und 100 °C betragen.

Gegebenenfalls kann die Belichtung in mehreren Schritten durchgeführt werden, beispielsweise in zwei bis acht Wiederholungen.

Vor und oder nach der Belichtung kann der behandelte Latex noch mit weiteren Prozesschemikalien, wie z.B. Alterungsschutzmittel, Stabilisatoren, Antiozonantien, Entschäumer, Farbstoffe, Pigmente, anorganische Füllstoffe, wie z.B. Kreide, versetzt werden.

Zur Herstellung eines Elastomerproduktes wird in der Folge in einem ersten Schritt eine erste Schicht aus einem Elastomer erzeugt, beispielsweise nach einem bekannten Tauchverfahren, und diese vorvernetzt, insbesondere photochemisch vorvernetzt. Nach zumindest einem Trocken- und/oder zumindest einem Waschschritt wird aus dem modifizierten Latex, der wie voranstehend funktionalisiert wurde, zumindest eine weitere Elastomerschicht auf die zuerst erzeugte Elastomerschicht aufgebracht, insbesondere aufgetaucht. Danach werden wiederum zumindest ein Trocken- und/oder zumindest ein Waschschritt ausgeführt.

Nach einer anderen Ausführungsvariante des Verfahrens ist vorgesehen, dass die Elastomeroberfläche zumindest bereichsweise mit einer Polymerschicht versehen wird, die kovalent an die Elastomeroberfläche angebunden wird.

Dazu wird in einem ersten Schritt wiederum die Funktionalisierung der Elastomeroberfläche mit zumindest einem Thiol durchgeführt, sodass an der Elastomeroberfläche zumindest eine Art der voranstehend genannten funktionellen Gruppen (-SH, -COOH, -NH₂, Epoxid, -NCO, - NCS) vorhanden ist. Die Ankopplung selbst kann dabei photochemisch oder thermisch erfolgen, wie diese bereits voranstehend erläutert wurde.

Die Polymerschicht kann beispielsweise aus einem Polyurethan oder einem Silikon oder aus einer Mischung aus SBR mit Silikon oder einem Acrylat oder einem Siloxan oder aus einem Polymer mit funktionellen Gruppen, insbesondere Alkene, Acrylate, Anhydride, Epoxide, Isocyanate, Isothiocyanate, Methacrylate, Thiole, um eine kovalente Bindung an die Elastomeroberfläche zu erzielen, hergestellt werden. Gegebenenfalls können die Polymere bzw. Monomere zur Bildung der Polymerschicht ebenfalls vorher funktionalisiert werden, insbesondere mit zumindest einer Art der erwähnten funktionellen Gruppen.

Die funktionellen Gruppen an dem Polymer können als Seitengruppen oder endständig vorliegen.

Bevorzugte Polymere sind Silikone, Polyurethane, Urethanacrylate, Acrylate, Polyisocyanate, Polyester Polyole, Vinylpolymere, Dien-Elastomere. Beispiele hierfür sind Desmophen^{®} 1652, Synthomer VL 11005, Desmolux^{®} XP 2740, Bayhydrol^{®} UV XP 264, Desmolux^{®} VP LS 2299, beziehbar bei Bayer bzw. Synthomer.

Aus den gegebenenfalls funktionalisierten Polymere oder Monomere bzw. Oligomere werden wiederum Suspensionen oder Lösungen erzeugt (zur Funktionalisierung kann das jeweilige Reagens dieser Suspension zugesetzt werden) oder es wird das Polymer als Reinsubstanz verwendet. Dem Polymer kann zumindest ein Emulgator und/oder zumindest ein Photoinitiator zugesetzt werden. Die Konzentrationen können entsprechend den voranstehenden Angaben gewählt werden.

Diese Suspension wird in der Folge auf ein insbesondere vorvernetztes, vorzugsweise photochemisch vorvernetztes, Elastomer aufgebracht, insbesondere aufgetaucht, gegebenenfalls getrocknet, und danach belichtet, wozu die voranstehend genannten Strahlungsquellen und Bestrahlungsparameter verwendet werden können.

Aufgebrachte Monomere bzw. Oligomere oder die Polymeren können nach der Anbindung an die Elastomeroberfläche auch noch vernetzt werden.

Prinzipiell besteht die Möglichkeit, dass die gesamte Oberfläche des funktionalisierten Elastomers mit zumindest einer weiteren chemischen Verbindung und/oder mit Feststoffpartikel und/oder mit der Polymerbeschichtung versehen wird.

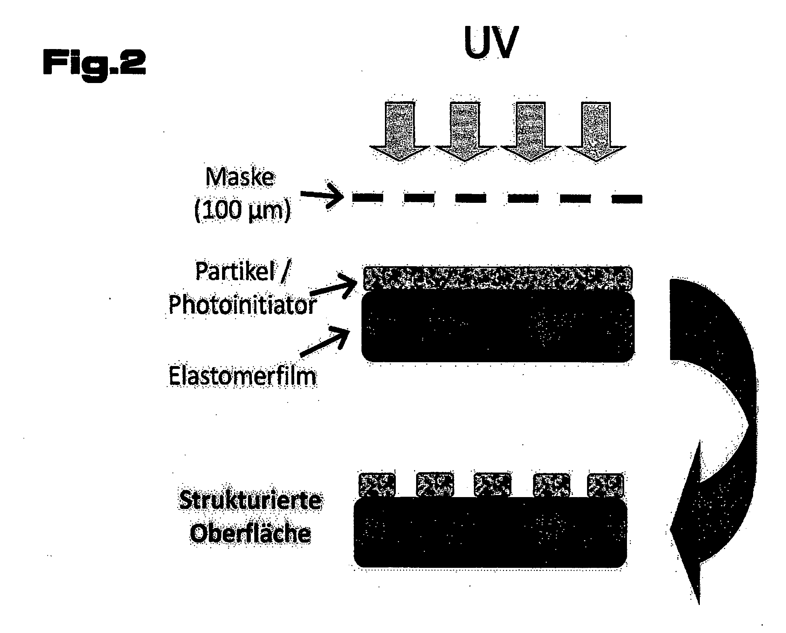

Gemäß einer Ausführungsvariante dazu kann jedoch vorgesehen sein, dass die zumindest eine weitere chemische Verbindung und/oder die Feststoffpartikel und/oder die Polymerbeschichtung lediglich in diskreten Bereichen an der Elastomeroberfläche ausgebildet bzw. angeordnet werden. Um dies zu erreichen, kann mit einer entsprechenden Maske der nicht weiter zu funktionalisierende Bereich der Elastomeroberfläche bei der Belichtung mit der Strahlenquelle abgedeckt werden, wie dies in Fig. 2 dargestellt ist, sodass in einem anschließenden Waschschritt die nicht photochemisch reagierten und damit nicht kovalent gebundenen Substanzen abgewaschen werden.

Nach einer nicht bevorzugten Ausführungsvariante dazu besteht die Möglichkeit, dass die zumindest eine weitere chemische Verbindung und/oder die Feststoffpartikel und/oder die Suspension für die Polymerbeschichtung bereits nur in diskreten Bereichen aufgetragen wird.

Die Maske kann eine mechanische Maske oder eine chemische Maske oder eine optische Maske sein. Unter einer chemischen Maske wird eine Substanz verstanden, die vor dem Auftragen der jeweiligen Suspensionen oder Emulsionen oder Lösung auf die nicht zu beschichtenden Bereiche aufgebracht wird, beispielsweise aufgestrichen.

Neben der reinen Oberflächenstrukturierung kann damit beispielsweise auch eine dauerhafte Anbringung von Informationen auf dem Elastomerprodukt, beispielsweise der Handschuhgröße bei Elastomerhandschuhen, erreicht werden.

Obwohl im Voranstehenden lediglich die zumindest teilweise, bevorzugt vollständige Absättigung der ungesättigten Kohlenstoff-Kohlenstoff Bindungen des Elastomers behandelt wurde, ist es prinzipiell auch möglich, wenngleich nicht bevorzugt, dass die ungesättigten Kohlenstoff-Kohlenstoff Bindungen innerhalb des Elastomers zumindest teilweise photochemisch mit zumindest einem Thiol gesättigt werden.

Mit Ausnahme der ersten beschriebenen Ausführungsvariante des Verfahrens wird bei allen anderen Verfahren nach der Erfindung eine Funktionalisierung der Funktionalisierung, d.h. der funktionalisierten Oberfläche des Elastomerhandschuhs, durchgeführt. Die infolge der ersten Funktionalisierung auf der Oberfläche angeordneten funktionellen Gruppen wirken dabei als Ankergruppen für die weitere Funktionalisierung.

Die photochemische Ankopplung des zumindest einen Thiols an die Oberfläche des Elastomerhandschuhs erfolgt nach der Thiol-En Reaktion

Mit dem Verfahren nach der Erfindung können Elastomerhandschuhe hergestellt werden, die eine bessere Gleitfähigkeit und eine bessere Alterungsbeständigkeit im Vergleich zu einem unbehandelten Elastomer aufweisen. Darüber hinaus können damit Eigenschaften, wie z.B. Benetzbarkeit, Polarität, Gleitreibung beeinflusst werden bzw. dem Elastomerprodukt völlig neue Eigenschaften verliehen werden, wie z.B. strukturierte Elastomeroberflächen, Geruch, Farbe, "Look and Feel". Je nach Wahl des Thiols können der Elastomeroberfläche polare oder unpolare Eigenschaften verliehen werden.

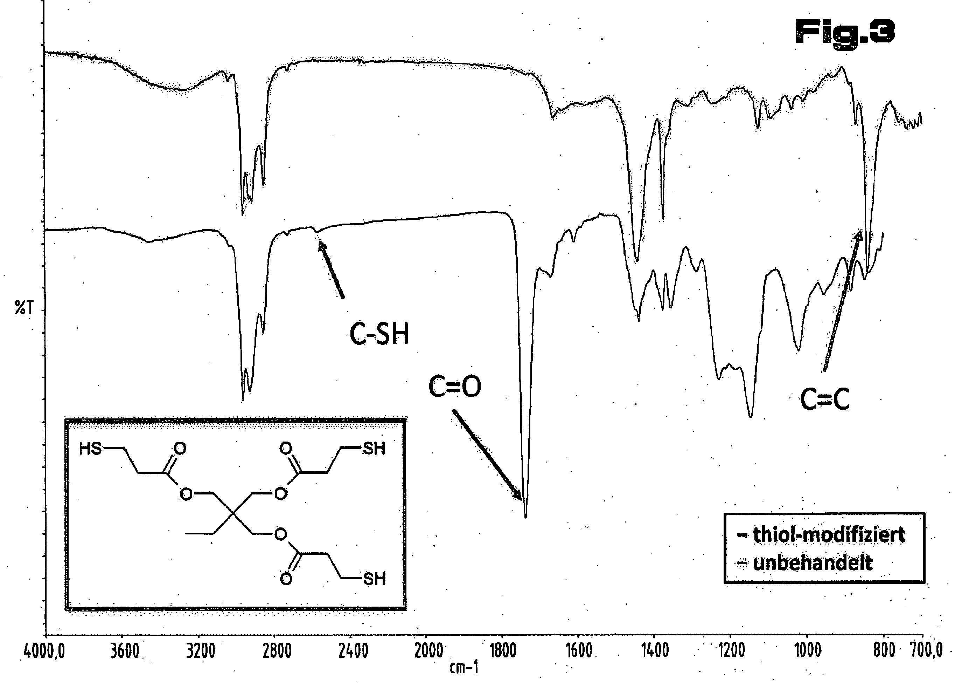

Zur Evaluierung der kovalenten Bindung zwischen dem Elastomer und dem Thiol wurde TriThiol als Modellsubstanz und ein Polyisopren gewählt. Es wurden Proben mittels Infrarotspektroskopie untersucht. Fig. 3 zeigt ein aufgenommenes Spektrum.

Aus den Ergebnissen der Infrarotspektroskopie kann geschlossen werden, dass der Einbau des TriThiols über eine kovalente Anbindung an die Elastomeroberfläche erfolgt, da die C=C Doppelbindungen (831cm-1) in Folge der Modifizierung abnehmen. Zusätzlich lassen sich bei den modifizierten Elastomeroberflächen IR-Banden bei 2430cm-1 und 1735 cm-1 nachweisen, die auf das TriThiol zurückzuführen sind.

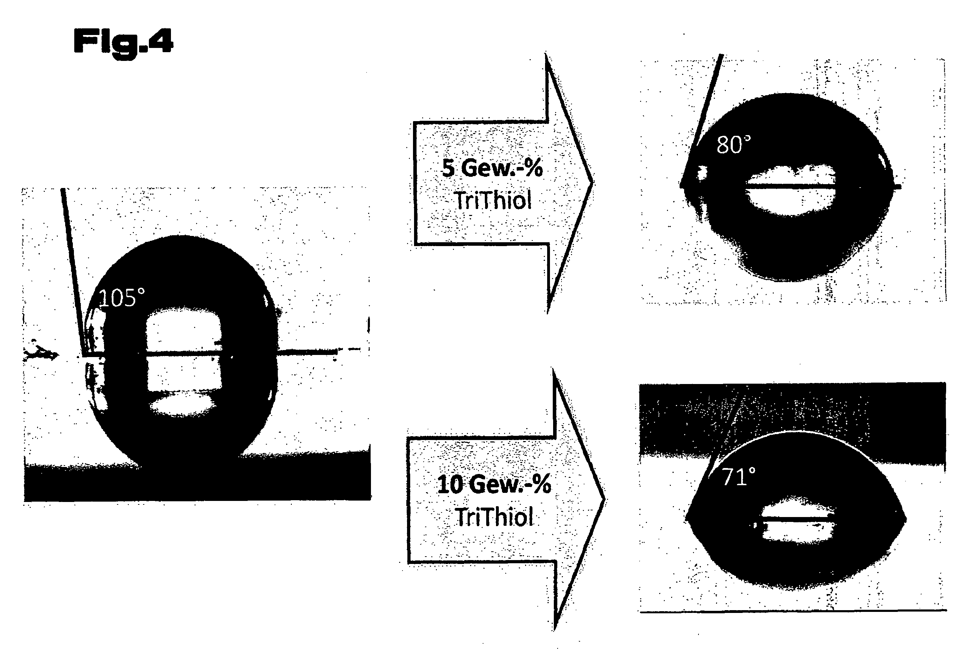

An von mit TriThiol modifizierten NR-Latexfilmen wurden weiter Kontaktwinkelmessungen durchgeführt. Das Ergebnis ist in Fig. 4 dargestellt.

Durch den Einbau des TriThiols wird sowohl der polare als auch der disperse Energieahteil der Oberflächenenergie der Polyisoprenoberfläche erhöht - d.h. die Benetzbarkeit und die Polarität der Oberfläche nehmen zu, wie dies aus folgender Tabelle entnommen werden kann.

| Thiol-Konzentration [Gew.-%] | Polarer Energieanteil [mN/m] | Disperser Energieanteil [mN/m] | Gesamte Oberflächenenergie [mN/m] |
|---|---|---|---|
| 0 | 1,0 | 34,6 | 35,6 |
| 1,5 | 4,7 | 34,5 | 39,2 |
| 5 | 3,6 | 36,2 | 39,8 |
| 10 | 2,7 | 43,9 | 46,8 |

Wie aus Fig. 4 ersichtlich ist, nimmt der Kontaktwinkel von 105 ° des unbehandelten NR-Latex auf 80 ° mit 5 Gew.-% TriThiol bzw. 71 ° mit 10 Gew.-% TriThiol ab.

Generell kann mit dem Verfahren nach der Erfindung eine Reduktion des Kontaktwinkels zu Wasser um zumindest 10 %, bezogen auf die unbehandelte Elastomeroberfläche, erreicht werden. Durch den Einsatz von N-Acetylcystein kann die Polarität der Elastomeroberfläche sogar beinahe verdoppelt werden (Reduktion von 105 ° unbehandelt NR-Latexfilm auf 54 ° bei 5 Gew.-% N-Acetylcystein).

Interessanterweise wurde festgestellt, dass der Kontaktwinkel bei Einsatz von 10 Gew.-% N-Acetylcystein wieder zunimmt (74 °).

Es wurde weiter festgestellt, dass durch die kovalente Anbindung von Feststoffpartikel die Polarität wieder sinkt (Modifizierung mit vinylfunktionalisierten SiO₂ Partikel: Wasserkontaktwinkel 91°).

Mit anderen Worten können also mit dem Verfahren der Erfindung Elastomere zur Verfügung gestellt werden, die "maßgeschneiderte" Polaritäten an der Oberfläche aufweisen. Damit ist auch die Reaktivität der Elastomeroberfläche entsprechend einstellbar.

Die Messungen wurden entsprechend Owens DK, Wendt RC. Estimation of the surface free energy of polymers. J Appl Polym Sci 1969;13(8):1741-1747 bzw. Rabel W. Wetting theory and its application to the study and use of the surface properties of polymers. Farbe und Lacke. 1971;77(10):997-1006 durchgeführt.

Die modifizierten Oberflächen wurden auch mittels Zetapotentialmessung charakterisiert. Bei nicht modifizierten Naturkautschukoberflächen liegt der isoelektrische Punkt (=0) bei 3,45, was auf eine schwach negativ geladene Oberfläche schließen lässt. Inerte Polymeroberflächen, wie z.B. Polypropylen, haben zwar einen isolektrischen Punkt (=0) von 3,8 - 4,1, aber die Proteine, Phospholipide und andere organische Begleitstoffe können bei der NR-Oberfläche zu einer Verschiebung in den sauren Bereich führen.

Durch die Funktionalisierung mit TriThiol (SH-funktionalisierte Oberfläche) wird ein isolelektrischer Punkt (IEP) von 3,0 erhalten, was auf eine stärker negativ geladene Oberfläche hinweist. Dieser Wert ist vergleichbar mit OH-funktionalisierten Oberflächen, die ebenfalls einen IEP im Bereich von 3,0 haben. Durch die kovalente Bindung der Vinyl-Partikel an die Mercaptogruppen bekommt der Film wieder die Eigenschaften einer inerten Oberfläche. Dies konnte mittels Zetapotentialmessung bestätigt werden, denn der IEP verschiebt sich von 3,0 auf 3,9. Die mit Vinyl-Partikeln funktionalisierte Probe wurde in einer Doppelbestimmung charakterisiert. Es wurde bei beiden Messungen ein deckungsgleicher IEP erhalten, was auf eine kovalente Bindung der Partikel hindeutet.

In weiterer Folge wurde mit Hilfe von tribologischen Messmethoden mit einem Lineartribometer entsprechend B. Bhushan, Modern tribology handbook. CLC-Press, Boca Raton, London, New York, Washington D.C. 2001, der Gleitreibungskoeffizient von Partikel modifizierten NR-Oberflächen bestimmt und mit den Eigenschaften von kommerziellen Operationshandschuhen verglichen. Die Ergebnisse in folgender Tabelle zeigen, dass die Gleitreibungseigenschaften von Partikel modifizierten Oberflächen im Bereich von gepuderten NR-Oberflächen liegen.

Vergleich der Gleitreibungskoeffizienten von ausgewählten NR-Oberflächen

| Probenbeschreibung | Gleitreibungskoeffizient |
|---|---|
| Stand der Technik Handschuh mit chlorierter Innenseite | m ∼ 0,31 |
| Stand der Technik Handschuh mit beschichteter Innenseite | m ∼ 0,22 |
| Stand der Technik Handschuh mit gepuderte Innenseite | m ∼ 0,50 |
| Vinyl-Partikel modifizierte NR-Oberfläche | m ∼ 0,55 - 0,77 |

Im Folgenden sind einige im Zuge der Erarbeitung der Erfindung durchgeführte, nicht beschränkend zu verstehende Beispiele angegeben.

Die für die Beispiele verwendeten Chemikalien sind in Tabelle 1 zusammengestellt.

**Tabelle 1: verwendete Materialien und Chemikalien**

| Chemikalie | Hersteller | Strukturformel, Spezifikation |
|---|---|---|
| Aktisil^{®} MM | Hofmann Mineral | Mercapto-modifizieite SiO₂-Patikel (d₅₀ = 2,2 µm) |
| Aktisil^{®} VM 56 | Hoffmann Mineral | Vinyl-modifizierte SiO₂-Partike (d₅₀ = 2,2 µm) |
| TWEEN^{®} 20 | Sigma Aldrich | 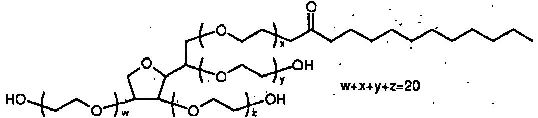 |
| Lucirin^{®} TPO-L | BASF | 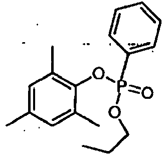 |
| Genocure^{®} DMHA | Rahn AG | 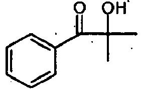 |
| Irgacure^{®} 2959 | Sigma Aldrich | 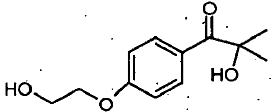 |
| N-Acetylcystein | Sigma Aldrich | 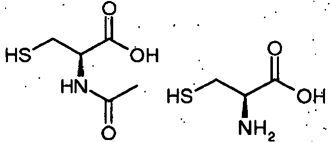 |
| L-Cystein | Sigma Aldrich | |
| Trimethylolpropantris-3-mercaptopropionat (TriThiol) | Bruno Bock Chemische Fabrik GmbH Co KG | 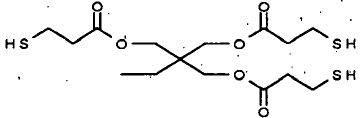 |
| Bayhydrol® UV XP 2649 | Bayer | Urethanacrylat-Dispersion |
| Bayhydrol® UV XP 2740 | Bayer | Acrylat-Dispersion |
| Styrolbutadien | | Redispergierbare Polymerdispersion |
| Poly(Mercaptopropyl) methylsiloxan | ABCR | |

### Beispiel 1: Modifizierung einer getrockneten Filmoberfläche, Ankopplung von trifunktionellen Thiolderivaten (Gerierierung von freien SH-Gruppen)

Bei der photochemischen Ankopplung von TriThiol via Thiol-En Reaktion werden nachfolgende Schritte durchgeführt:
- Herstellen einer wässrigen Emulsion mit 10 Gew.-% Trithiol, 1,1 Gew.-% TWEEN 20 und 1 Gew.-% Lucirin TPO L
- Dispergieren der Emulsion mittels Dispergiergerät (Ultraturax) für 5 min bei Räumtemperatur
- Fixieren eines UV vorvernetzten NR-Latexfilms in einer Petrischale mit Klebestreifen
- Übergießen des Elastomerfilms mit der wässrigen Emulsion
- Film nach 10 min aus der Petrischale entnehmen
- Trocknen der Probe für 1 min bei 100 °C
- Probe mit Hg-Strahler (Fusion UV) belichten (Parameter: Siehe Tabelle 2)

**Tabelle 2: Geräteparameter der Belichtungsanlage (Fusion UV) für die SH-Funktionalisierung**

| Geräteparameter | Einstellungen |
|---|---|
| Durchläufe | 1 - 4 |
| Lampentyp | Quecksilberstrahler (nicht dotiert und Ga-dotiert) |
| Lampenleistung | 40 % - 60% |
| Förderbandgeschwindigkeit | 3,5 m/min - 6 m/min |
| Bestrahlungsdosis | 0,5 J/cm²- 25 J/cm² |

- 10 min in H₂O_{deion}. und 10 min in Ethanol unter Rühren (Magnetrührer) waschen
- Funktionalisierte Probe anschließend für 10 min bei 100°C trocknen

### Beispiel 2: Modifizierung einer getrockneten Filmoberfläche, Ankopplung von monofunktionellen Thiolderivaten (Generierung von freien NH₂- und COOH-Gruppen)

Bei der photochemischen Ankopplung von N-Acetylcystein bzw. Cystein via Thiol-En Reaktion werden nachfolgende Schritte durchgeführt:
- Herstellen einer wässrigen Emulsion mit 1 - 10 Gew.-% N-Acetylcystein bzw. Cystein, 1,1 Gew.-% TWEEN 20 und 1 Gew.-% Lucirin TPO L

Die weiteren Schritte werden analog zu Beispiel 1 durchgeführt.

### Beispiel 3: Photochemische Modifizierung der flüssigen Phase

Bei der photochemischen Ankopplung von N-Acetylcystein bzw. Cystein via Thiol-En Reaktion werden nachfolgende Schritte durchgeführt:
- Prozesschemikalien (1 - 2 phr Irgacure, 1 - 20 phr L-Cystein bzw. N-Acetylcystein) unter Rühren bei erhöhter Temperatur in deionisiertem Wasser lösen. Wassermenge so bemessen, dass sich nach NR-Latexzugabe ein Feststoffgehalt von 30 %dic. einstellt.
- Prozesschemikalienlösung einem vorvulkanisierten NR-Latex zugeben
- Rühren der Mischung bei RT für 2h mit Magnetrührer
- 16 ml der Mischung in eine Glaspetrischale überführen (1mm Schichtdicke)
- Mischung mit Hg-Strahler bzw. Ga dotiertem Hg-Strahler (Fusion UV) belichten (Parameter: Siehe Tabelle 3)

**Tabelle 3: Geräteparameter der Belichtungsanlage (Fusion UV) für die Modifikation von Latex**

| Geräteparameter | Einstellungen |
|---|---|
| Durchläufe | 1-4 |
| Lampentyp | Quecksilberstrahler (nicht dotiert und Ga-dotiert) |
| Lampenleistung | 40% - 60% |
| Förderbandgeschwindigkeit | 3,5 m/min - 6 m/min |
| Bestrahlungsdosis | 0,5 J/cm² - 25 J/cm² |

- Alternativ kann die UV-Belichtung auch im Fallfilmreaktor durchgeführt werden
- Mischung mit 0,5 phr Ralox (Alterungsschutzmittel) versetzen
- Rühren der Mischung bei RT für 2h mit Magnetrührer

Die entsprechenden Latexfilme werden in einer Zweischichttauchung hergestellt:
- Auftauchen eines vorvernetzten NR-Latex auf eine Porzellanform (20s bei RT)
- 0 s - 15 s trocknen bei 120 °C
- Auftauchen des modifizierten NR-Latex (30 s bei Raumtemperatur RT (ca. 20 °C)
- 60 s - 90 s trocknen bei 120 °C
- Waschen in H₂O_{deion}. bei 80 °C für 60 s
- Trocknen für 15 min bei 120 °C
- Waschen in H₂O_{deion}. bei 80 °C für 60 s
- Trocknen für 5 min bei 120°C

### Beispiel 4: Photochemische Ankopplung von anorganischen Partikeln, Durchführung in wässrigen Systemen

Im Zuge der photochemischen Ankopplung von anorganischen SiO₂-Makropartikeln werden nachfolgende Prozessschritte durchgeführt:
- Herstellen einer wässrigen Suspension mit 0,015 Gew.-% - 0,5 Gew.-% Vinyl-bzw. - SH modifizierter SiO₂-Makropartikel, 0,15 Gew.-% - 0,7 Gew.-% TWEEN 20 und 1,7 Gew.-% Genocure DMHA
- Dispergieren der Suspension im Ultraschallbad für 10 min - 20 min bei Raumtemperatur
- Fixieren eines -SH-funktionalisierten NR-Latexfilms (Siehe Beispiel 1) in einer Petrischale
- Übergießen des Elastomerfilms mit der wässrigen Suspension
- Film nach 2 min aus der Petrischale entnehmen
- Trocknen der Probe für 10 min bei 70 °C
- Probe mit Hg-Strahler (Fusion UV) belichten (Siehe Tabelle 3)
- Belichteter Film für 16 h bei Raumtemperatur in Wasser waschen
- Film für 10 min - 15 min bei 70 °C trocknen

### Beispiel 5: Photochemische Ankopplung von anorganischen Partikeln, Durchführung in organischen Lösungsmitteln

- Herstellen einer Suspension mit 0,015 Gew.-% - 0,2 Gew.-% Vinyl- bzw. -SH modifizierter SiO₂-Makropartikel und 1,7 Gew.-% Lucirin TPO-L in Toluol
- Dispergieren der Suspension im Ultraschallbad für 10 min bei Raumtemperatur
- Übergießen des in einer Petrischale liegenden Elastomerfilms mit der Suspension. Aufschwimmen des Films mittels Pinzette verhindern.
- Film nach 2 min aus der Petrischale entnehmen
- Trocknen der Probe für 10 min bei 70 °C
- Probe mit Hg-Strahler (Fusion UV) belichten (Siehe Tabelle 3)
- Belichteter Film für 16 h bei Raumtemperatur in Toluol waschen
- Film für 10 min - 45 min bei 70 °C trocknen

### Beispiel 6: Thermische Ankopplung von anorganischen Partikeln, Durchführung in wässrigen Systemen

Im Zuge der thermischen Ankopplung von anorganischen SiO₂-Makropartikeln werden nachfolgende Prozessschritte durchgeführt:
- Herstellen einer wässrigen Suspension mit 0,015 Gew.-% - 0,5 Gew.-% Epoxy-modifizierter SiO₂-Makropartikel
- Dispergieren der Suspension mit Dispergiergerät (Ultraturax) für 10 min bei Raumtemperatur und anschließend im Ultraschallbad für 10 min bei Raumtemperatur
- Fixieren eines SH-funktionalisierten NR-Latexfilms (siehe voranstehend) in einer Petrischale
- Übergießen des Elastomerfilms mit der wässrigen Suspension
- Film nach 2 min aus der Petrischale entnehmen
- Trocknen der Probe für 10 min - 900 min bei 40 °C - 100°C
- Film für 16 h bei Raumtemperatur in Wasser waschen
- Film für 10 min - 45 min bei 70 °C trocknen

### Beispiel 7: Thermische Ankopplung von anorganischen Partikeln, Durchführung in organischen Lösungsmitteln

- Herstellen einer Suspension mit 0,015 Gew.-% - 0,2 Gew.-% - Epoxy-modifizierter SiO₂-Makropartikel in Toluol
- Dispergieren der Suspension im Ultraschallbad für 10 min bei Raumtemperatur
- Übergießen des Elastomerfilms mit der wässrigen Suspension. Aufschwimmen des Films mittels Pinzette verhindern
- Film nach 2 min aus der Petrischale entnehmen
- Trocknen der Probe für 10 min - 900 min bei 40 °C - 100°C
- Film für 16 h bei Raumtemperatur in Toluol waschen
- Film für 10 min - 15 min bei 70 °C trocknen

### Beispiel 8: Photochemische Ankopplung von Polymerbeschichtungen

Im Zuge der photochemischen Ankopplung von ausgewählten Polymerbeschichtungen werden nachfolgende Prozessschritte durchgeführt:
- Die Polymerbeschichtungen:
   a) Bayhydrol^{®} UV XP 2649 (Urethanacrylat-Dispersion)
   b) Redispergierte Styrolbutadien-Dispersion (40 Gew.-% in H₂O_{deion.})
   c) Bayhydrol^{®} UV XP 2740 (Acrylat-Dispersion)
   d) Poly(mercaptopropyl)methylsiloxan
   werden mit 1 Gew.-% Lucirin TPO L und wahlweise mit 1 Gew.-% - 5 Gew.-% TriThiol versetzt (emulgiert mit 1,1 Gew.-% Tween 20 in H₂O_{deion.}) und mit einer Dispergiereinheit (Ultraturax) dispergiert.
- Die Beschichtungen werden auf vorvernetzten und SH-funktionalisierten (Siehe Beispiel 1) NR-Latexfilmen aufgetaucht (15 s - 45 s bei Raumtemperatur)
- Trocknen der Beschichtungen für 15 min bei 70°C
- Probe mit Hg-Strahler (Fusion UV) belichten (Parameter: Siehe Tabelle 4)

**Tabelle 4: Geräteparameter der Belichtungsanlage (Fusion UV) für die Ankopplung von Polymerbeschichtungen**

| Geräteparameter | Einstellungen |
|---|---|
| Durchläufe | 1-3 |
| Lampentyp | Quecksilberstrahler (nicht dotiert) |
| Lampenleistung | 30%-40% |
| Förderbandgeschwindigkeit | 3 m/min - 6 m/min |
| Bestrahlungsdosis | 0,5 J/cm²-25 J/cm² |

### Beispiel 9: Thermische Ankopplung von Polymerbeschichtungen

Zur thermischen Ankopplung von Polymerbeschichtungen wurden die in Tabelle 5 zusammengefassten Kopplungsreaktionen von Polymerbeschichtungen an photochemisch modifizierter Oberfläche durchgeführt.

**Tabelle 5: Thermische Ankopplung von Polymerbeschichtungen auf photochemisch modifizierter Elastomeroberfläche - Oberfläche im getrockneten Zustand modifiziert**

| NR-Oberfläche photochemisch modifiziert | Polymerbeschichtung thermisch angekoppelt |
|---|---|
| | Substanzklasse |
| -SH | Polymere mit Epoxidgruppen |
| -SH | Polymere mit Mercaptogruppen |
| -COOH | Polymere mit Epoxidgruppen |

Die Ausfuhrungsbeispiele zeigen mögliche Ausführungsvarianten der Erfindung.

## Patentansprüche

1. Verfahren zur Modifizierung der Oberfläche eines Elastomerhandschuhs mit ungesättigten Kohlenstoff-Kohlenstoff-Bindungen, **dadurch gekennzeichnet, dass** die ungesättigten Kohlenstoff-Kohlenstoff-Bindungen im Bereich der Oberfläche zumindest teilweise durch eine photochemische Reaktion mit zumindest einem Thiol gesättigt werden oder indem auf die Oberfläche des Elastomerhandschuhs zumindest bereichsweise eine Schicht aus einem Latex aufgetaucht oder aufgebracht wird, dessen ungesättigten Köhlenstoff-Kohlenstoff-Bindungen im Bereich seiner Oberfläche zumindest teilweise durch eine photochemische Reaktion mit zumindest einem Thiol gesättigt sind.

2. Verfahren nach Anspruch 1, **dadurch gekennzeichnet, dass** durch die Reaktion mit dem zumindest einem Thiol freie -SH Gruppen auf der Oberfläche des Elastomerhandschuhs erzeugt werden.

3. Verfahren nach Anspruch 1 oder 2, **dadurch gekennzeichnet, dass** als Thiol eine chemische Verbindung verwendet wird, die ausgewählt ist aus einer Gruppe umfassend Trimethylolpropan-tris-3-mercaptopropionat, Pentaerythritoltetramercaptoacetat, Trimethylolpropantrimercaptoacetat, Trimethylolpropanetri-3-mercaptopropionat, Pentaerythritoltetra-3-mercaptopropionat, Propylenglycol-3-mercaptopropionat, ethoxyliertes Trimethylolpropantri-3-mercaptopropionat, Polyol-3-mercaptopropionat, Polyester-3-mercaptopropionat.

4. Verfahren nach einem der Ansprüche 1 bis 3, **dadurch gekennzeichnet, dass** der Elastomerhandschuh vorvernetzt eingesetzt wird.

5. Verfahren nach Anspruch 4, **dadurch gekennzeichnet, dass** die Vorvernetzung photochemisch durchgeführt wird.

6. Verfahren nach einem der Ansprüche 1 bis 4, **dadurch gekennzeichnet, dass** die Reaktion mit dem zumindest einem Thiol an einer festen Oberfläche des Elastomerhandschuhs ausgeführt wird.

7. Verfahren nach einem der Ansprüche 2 bis 7, **dadurch gekennzeichnet, dass** die freien-SH Gruppen mit zumindest einer weiteren chemischen Verbindung und/oder mit Feststoffpartikel umgesetzt werden.

8. Verfahren nach Anspruch 7, **dadurch gekennzeichnet, dass** die weitere chemische Verbindungen ausgewählt werden aus einer Gruppe umfassend Alkene, Acrylate, Anhydride, Epoxide, Isocyanate, Isothiocyanate, Methacrylate, Thiole.

9. Verfahren nach Anspruch 7, **dadurch gekennzeichnet, dass** die Feststoffpartikel durch anorganische Partikel gebildet sind.

10. Verfahren nach Anspruch 7 oder 9, **dadurch gekennzeichnet, dass** die Feststoffpartikel vor der Reaktion mit den -SH Gruppen oberflächlich funktionalisiert werden.

11. Verfahren nach Anspruch 10, **dadurch gekennzeichnet, dass** die Funktionalisierung der Feststoffpartikel durch Erzeugung von freien Epoxygruppen, Mercaptogruppen, Acrylatgruppen, Anhydridgruppen, Isocyanatgruppen, Isothiocyanatgruppen, Methacrylatgruppen, Vinylgruppen, an der Oberfläche der Feststoffpartikel durchgeführt wird.

12. Verfahren nach Anspruch 10, **dadurch gekennzeichnet, dass** die Funktionalisierung der Feststoffpartikel mit zumindest einer chemischen Verbindung durchgeführt wird, die ausgewählt ist aus einer Gruppe umfassend Silane, Siloxane und Carbonsäuren mit funktionellen Gruppen, wie Acrylat-, Anhydrid-, Epoxy-, Isocyanat-, Isothiocyanat-, Mercapto-, Methacrylat-, Vinylgruppen. Beispiele hierfür sind Vinyltriethoxysilan, (3-Glycidoxypropyl)trimethoxysilan, 3-(Triethoxysilyl)propylsuccinic Anhydride, Mercaptopropyltrimethoxysilan.

13. Verfahren nach einem der Ansprüche 7 bis 11, **dadurch gekennzeichnet, dass** adhäsiv gebundene Partikel von der Oberfläche des Elastomerhandschuhs entfernt werden.

14. Verfahren nach einem der Ansprüche 1 bis 5 oder 6 bis 12, **dadurch gekennzeichnet, dass** die photochemische Reaktion mit zumindest einem Thiol an einem Latex in flüssiger Phase durchgeführt wird und der Latex danach auf einen, insbesondere vorvernetzten, Latexfilm aufgetaucht wird.

15. Verfahren nach einem der Ansprüche 1 bis 13, **dadurch gekennzeichnet, dass** die Oberflächenmodifizierung in diskreten Bereichen des Elastomerhandschuhs durchgeführt wird.

16. Verfahren nach einem der Ansprüche 1 bis 14, **dadurch gekennzeichnet, dass** auf die Oberfläche des Elastomerhandschuhs eine Polymerbeschichtung aufgebracht wird.

17. Elastomerhandschuh mit ungesättigten Kohlenstoff-Kohlenstoff-Bindungen und mit einer Oberfläche, **dadurch gekennzeichnet, dass** die ungesättigten Kohlenstoff-Kohlenstoff-Bindungen an der Oberfläche zumindest teilweise durch einfach oder mehrfach funktionelle -SR Gruppen funktionalisiert sind, wobei R für zumindest ein Element steht, das ausgewählt ist aus einer Gruppe umfassend H, Vinylverbindungen, Acrylate, Amine, Aminosäuren (Cystein), acetylierte Aminosäuren (N-Acetylcystein), Anhydride, Carbonsäuren, Ether, Epoxide, Isocyanate, Isothiocyanate Methacrylate, Silane, Siloxane, Feststoffpartikel.

18. Elastomerhandschuh nach Anspruch 17, **dadurch gekennzeichnet, dass** über die -SR Gruppen zumindest bereichsweise eine Polymerbeschichtung kovalent mit dem Elastomerhandschuh verbunden ist.

19. Elastomerhandschuh nach Anspruch 17 oder 18, **dadurch gekennzeichnet, dass** dieser zumindest zweischichtig ausgebildet ist.

20. Elastomerhandschuh nach einem der Ansprüche 17 bis 19, **dadurch gekennzeichnet, dass** die -SR Gruppen nur in diskreten Bereichen der Oberfläche angeordnet sind.

## Claims

1. Method of modifying the surface of an elastomer glove with unsaturated carbon-carbon bonds **characterized in that** the unsaturated carbon-carbon bonds are at least partially saturated in the region of the surface by means of a photo-chemical reaction with at least one thiol or by applying or dipping to apply a layer of latex to at least certain regions of the surface of the elastomer glove, the unsaturated carbon-carbon bonds of which in the region of its surface are at least partially saturated by a photochemical reaction with at least one thiol.

2. Method according to claim 1, **characterized in that** free -SH groups are created on the surface of the elastomer glove due to the reaction with the at least one thiol.

3. Method according to claim 1 or 2, **characterized in that** a chemical compound selected from a group comprising trimethylolpropane-tris-3-mercaptopropionate, pentaerythritol te-tramercaptoacetate, trimethylol propane trimercaptoacetate, trimethylol propane tri-3-mercaptopropionate, pentaerythritol tetra-3-mercaptopropionate, propylene glycol-3-mercaptopropionate, ethoxylated trimethylol propane tri-3-mercaptopropionate, polyol-3-mercaptopropionate, polyester-3-mercaptopropionate is used as thiol.

4. Method according to one of claims 1 to 3, **characterized in that** the elastomer glove is pre-crosslinked.

5. Method according to claim 4, **characterized in that** the pre-crosslinking is implemented photochemically.

6. Method according to one of claims 1 to 4, **characterized in that** the reaction with the at least one thiol is implemented on a solid surface of the elastomer glove.

7. Method according to one of claims 2 to 6, **characterized in that** the free -SH groups are reacted with at least one other chemical compound and/or with solid particles.

8. Method according to claim 7, **characterized in that** the other chemical compounds are selected from a group comprising alkenes, acrylates, anhydrides, epoxides, isocyanates, isothiocyanates, methacrylates, thiols.

9. Method according to claim 7, **characterized in that** the solid particles are inorganic particles.

10. Method according to claim 7 or 9, **characterized in that** the surface of the solid particles is functionalized prior to the reaction with the -SH groups.

11. Method according to claim 10, **characterized in that** the solid particles are functionalized by creating free epoxy groups, mercapto groups, acrylate groups, anhydride groups, isocyanate groups, isothiocyanate groups, methacrylate groups, vinyl groups, on the surface of the solid particles.

12. Method according to claim 10, **characterized in that** the functionalization of the solid particles is implemented with at least one chemical compound selected from a group comprising silanes, siloxanes and carboxylic acids with functional groups, such as acrylate, anhydride, epoxy, isocyanate, isothiocyanate, mercapto, methacrylate, vinyl groups, examples of which are vinyl triethoxysilane, (3-glycidoxypropyl)tumethoxysilane, 3-(tuethoxysilyl)propylsuccinic anhydrides, mercaptopropyl trimethoxysilane.

13. Method according to one of claims 7 to 11, **characterized in that** adhesively bonded particles are removed from the surface of the elastomer glove.

14. Method according to one of claims 1 to 5 or 6 to 12, **characterized in that** the photochemical reaction is implemented with at least one thiol on a latex in liquid phase and the latex is then applied to a latex film, which is pre-crosslinked in particular.

15. Method according to one of claims 1 to 13, **characterized in that** the surface modification is implemented in discrete regions of the elastomer glove.

16. Method according to one of claims 1 to 14, **characterized in that** a polymer coating is applied to the surface of the elastomer glove.

17. Elastomer glove with unsaturated carbon-carbon bonds and with a surface, **characterized in that** the unsaturated carbon-carbon bonds on the surface are at least partially functionalized by single- or multi-functional -SR groups, where R stands for at least one element selected from a group comprising H, vinyl compounds, acrylates, amines, amino acids (cysteine), acetylated amino acids (N-acetyl cysteine), anhydrides, carboxylic acids, ethers, epoxides, isocyanates, isothiocyanates methacrylates, silanes, siloxanes, solid particles.

18. Elastomer glove according to claim 17, **characterized in that** a polymer coating is covalently bonded to at least certain regions of the elastomer product by means of the -SR groups.

19. Elastomer glove according to claim 17 or 18, **characterized in that** it comprises at least two layers.

20. Method according to one of claims 17 to 19, **characterized in that** the -SR groups are disposed in only discrete regions of the surface.

## Revendications

1. Procédé destiné à la modification de la surface d'un gant en élastomère ayant des liaisons carbone-carbone insaturées, **caractérisé en ce que** les liaisons carbone-carbone insaturées dans la zone de la surface sont saturées au moins partiellement par une réaction photochimique avec au moins un thiol ou en ajoutant par trempage ou en appliquant sur la surface du gant en élastomère, au moins sur certaines parties, une couche d'un latex dont les liaisons carbone-carbone insaturées dans la zone de la surface sont saturées au moins partiellement par une réaction photochimique avec au moins un thiol.

2. Procédé selon la revendication 1, **caractérisé en ce que** par la réaction avec le ou les thiols, des groupes -SH libres sont produits sur la surface du gant en élastomère.

3. Procédé selon la revendication 1 ou 2, **caractérisé en ce que** l'on utilise comme thiol un produit chimique choisi dans un groupe comprenant tris-3-mercaptopropionate de triméthylolpropane, tétramercaptoacétate de pentaérythritol, trimercaptoacétate de triméthylolpropane, tri-3-mercaptopropionate de triméthylolpropane, tétra-3-mercaptopropionate de pentaérythritol, 3-mercaptopropionate de propylèneglycole, tri-3-mercaptopropionate éthoxylé de triméthylolpropane, 3-mercaptopropionate d'un polyol, 3-mercaptopropionate de polyester.

4. Procédé selon l'une des revendications 1 à 3, **caractérisé en ce que** le gant élastomère est pré-réticulé.

5. Procédé selon la revendication 4, **caractérisé en ce que** la pré-réticulation est mise en oeuvre par voie photochimique.

6. Procédé selon l'une des revendications 1 à 4, **caractérisé en ce que** la réaction avec le ou les thiols est mise en oeuvre sur une surface solide du gant en élastomère.

7. Procédé selon l'une des revendications 2 à 6, **caractérisé en ce que** les groupes -SH libres sont mis en réaction avec au moins un autre produit chimique et/ou avec des particules de matière solide.

8. Procédé selon la revendication 7, **caractérisé en ce que** le produit chimique supplémentaire est choisi dans un groupe comprenant des alkènes, des acrylates, des anhydrides, des époxides, des isocyanates, des isothiocyanates, des méthacrylates, des thiols.

9. Procédé selon la revendication 7, **caractérisé en ce que** les particules de matière solide sont formées par des particules anorganiques.

10. Procédé selon la revendication 7 ou 9, **caractérisé en ce que**, avant la réaction avec les groupes -SH, les particules de matière solide sont fonctionnalisées à leur surface.

11. Procédé selon la revendication 10, **caractérisé en ce que** la fonctionnalisation des particules de matière solide est effectuée sur la surface des particules de matière solide par production de groupes libres époxy, mercapto, acrylate, anhydride, isocyanate, isothiocyanate, méthacrylate, vinyle.

12. Procédé selon la revendication 10, **caractérisé en ce que** la fonctionnalisation des particules de matière solide est effectuée avec au moins un produit chimique choisi dans un groupe comprenant des silanes, des siloxanes et des acides de carboxyliques ayant des groupes fonctionnels tels que des groupes acrylate, anhydride, époxyde, isocyanate, isothiocyanate, mercapto, méthacrylate, vinyle. Des exemples étant triéthoxysilane de vinyle, triméthoxysilane de 3-glycidoxypropyle, anhydride succinique de 3-(triethoxysilyle)propyle, triméthoxysilane de mercaptopropyle.

13. Procédé selon l'une des revendications 7 à 11, **caractérisé en ce que** des particules liées par adhésion sont enlevés de la surface du gant en élastomère.

14. Procédé selon l'une des revendications 1 à 5 ou 6 à 12, **caractérisé en ce que** la réaction photochimique avec le ou les thiols est effectuée avec un latex en phase liquide et **en ce que** le latex est appliqué ensuite par trempage sur un film en latex notamment pré-réticulé.

15. Procédé selon l'une des revendications 1 à 13, **caractérisé en ce que** la modification de la surface est effectuée dans des zones discrètes du gant en élastomère.

16. Procédé selon l'une des revendications 1 à 14, **caractérisé en ce qu'**un revêtement en polymère est appliqué sur la surface du gant en élastomère.

17. Gant en élastomère ayant des liaisons insaturées carbone-carbone et avec une surface, **caractérisé en ce que** les liaisons insaturées carbone-carbone sont fonctionnalisées à la surface au moins partiellement par des groupes -SR à fonction unique ou plurielle, R représentant au moins un élément choisi dans un groupe comprenant H, des liaisons vinyle, des acrylates, des amines, des acides aminés (Cystéine), des acides aminés acétylées (cystéine de N-acétyle), des anhydrides, des acides carboxyliques, des éthers, des époxides, des isocyanates, des isothiocyanates, des méthacrylates, des silanes, des siloxanes, des particules de matière solide.

18. Gant en élastomère selon la revendication 17, **caractérisé en ce qu'**un revêtement en polymère est lié de manière covalente, par les groupes -SR, au moins par zones, au gant en élastomère.

19. Gant en élastomère selon la revendication 17 ou 18, **caractérisé en ce qu'**il est formé en au moins deux couches.

20. Gant selon l'une des revendications 17 à 19, **caractérisé en ce que** les groupes -SR sont disposés uniquement dans des zones discrètes de la surface.
